# EUROPEAN PATENT APPLICATION

(11) **EP 1 669 100 A1**
(43) Date of publication of application: **14.06.2006**
(21) Application number: 04106525.1
(22) Date of filing: 13.12.2004
(51) Int. Cl.: A61M 37/00

(54) **Micro-needle**

(71) Applicant: DEBIOTECH S.A., 1004 Lausanne (CH)
(72) Inventor: Neftel, Frédéric, Dr., 1006, Lausanne (CH); Schneider, Vincent, 1800, Vevey (CH); Schneeberger, Niklaus, 1009, Pully (CH)
(74) Representative: Besse, François

(57) **Abstract**

Hollow out-of-wafer-plane micro needle (1) comprising a support member (2), a closed pointed tip portion (3) and at least one side opening (4) in the needle body communicating with the needle lumen (5),
characterized by the fact that it comprises at least one barbed element (6) protruding from the needle body.

## Description

### Field of invention

The present invention relates to hollow out-of-wafer-plane micro needles having at least on side opening in the shaft. These needles are well suited for transdermal microfluidic applications, e. g. drug-or vaccine delivery.

### State of the art

Micro needles corresponding to the above cited definition are disclosed in international patent application WO 03/015860 A1.
One of the problems occuring with such needles is to maintain them fixed on the skin.

### Summary of the invention

The present invention offers an improvement with respect to the object disclosed in WO 03/015860 A1, which content is incorporated by reference.
To this effect, the micro needle according to the invention is provided with at least one barbed element protruding from the needle body.

In a first embodiment the barbed element is located between the support member and the side opening.

In another embodiment the barbed element is located between the needle distal end and the side opening.

Preferably the element is an annular flange surrounding the needle body.

Advantageously the barbed element is tapered towards the needle distal end and may not be tapered towards the needle proximal end.

In another embodiment the needle the general shape of a cross exhibiting a plurality of wings, and wherein said side opening is located in the corners of the cross where said wings connect to each other.

In another embodiment the needle has several side openings.

The invention also relates to a plate for transdermal transfer of liquid comprising an array of micro needles as previously defined which are provided on a support member.

The needle according to the invention is obtained according to
a process comprising the following steps :
- providing a wafer comprised of an etchable material and having a front side and a back side,
- making a blind hole in said wafer from its back side,
- providing a mask on the front side of the wafer such that the vertical projection of said mask at least partially covers the extension of said hole,
- performing a first isotropic etch under said mask to remove wafer material,
- anisotropically etching the wafer to form a protruding structure,
- performing a second isotropic etch on said protruding structure to expose the blind hole and form the needle distal end,
- anistropically etching the protruding structure to form a barbed element having a greater diameter than the diameter of the needle distal end,
- performing a wet oxidation on the needle distal end and on the barbed element followed by a dry oxide etch,
- anistropically etching the protruding structure to form a needle shaft,
- performing a third isotropic etch to form a shaft of smaller diameter than the diameter of the barbed element,
- performing a wet oxidation and a BHF cleaning.

The invention also relates to a process for obtaining an hollow out-of-wafer-plane micro needle characterized by the fact that it comprises the following steps :
- providing a wafer comprised of an etchable material and having a front side and a back side,
- making a blind hole in said wafer from its back side,
- providing a mask on the front side of the wafer such that the vertical projection of said mask at least partially covers the extension of said hole,
- performing a first isotropic etch under said mask to remove wafer material,
- anisotropically etching the wafer to form a protruding structure,
- performing a second isotropic etch on said protruding structure to expose the blind hole and form the needle distal end,
- anistropically etching the protruding structure to form a barbed element having a greater diameter than the diameter of the needle distal end,
- performing a wet oxidation on the needle distal end and on the barbed element followed by a dry oxide etch,
- anistropically etching the protruding structure to form a needle shaft,
- performing a third isotropic etch to form a shaft of smaller diameter than the diameter of the barbed element,
- performing a wet oxidation and a BHF cleaning.

### Detailed Description

The invention will be better understood with the support of examples illustrated by the following figures :

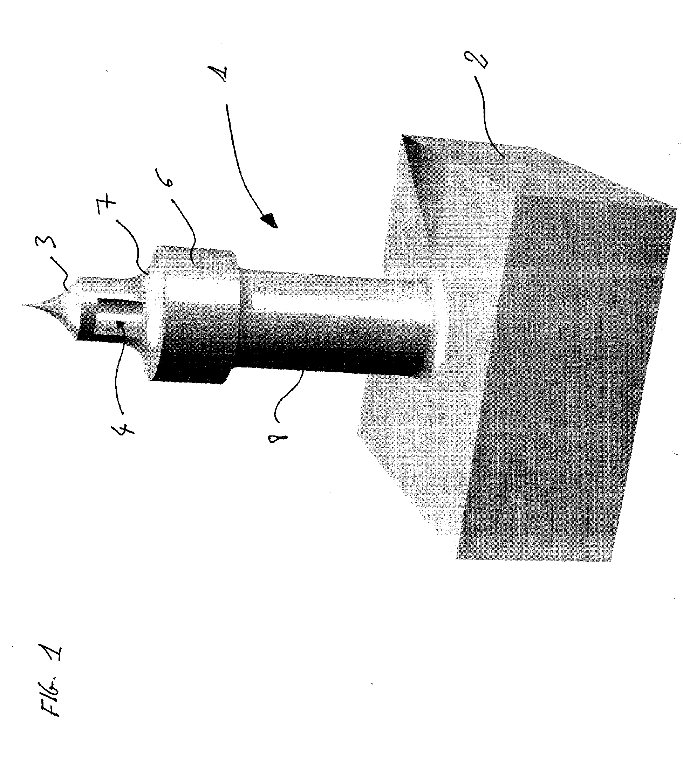

Figure 1 shows a first embodiment of a micro needle according to the invention.

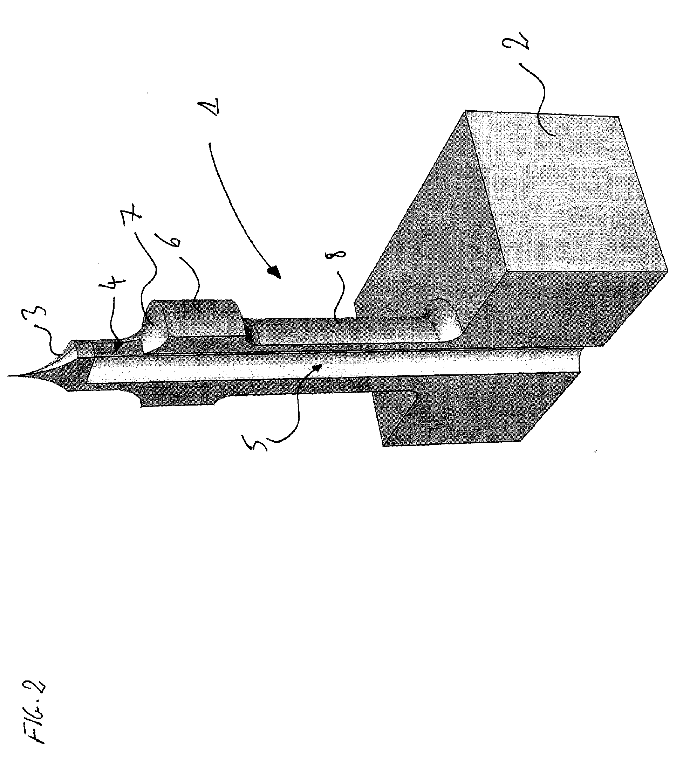

Figure 2 shows a section of the embodiment of figure 1.

The illustrated needle 1 is protruding from a support member 2 . Preferably the needle 1 and the support element 2 are made of silicon.
The needle 1 has a closed pointed tip portion 3 under which is a side opening 4.
An annular barbed element 6 is located bellow the side opening 4.
The upper portion 7 of the barbed element 6 is tapered in order to facilitate the needle penetration in the skin.
Inversely, the bottom portion of the barbed element 6 is not tapered in order to insure a proper fixation of the needle in the skin.
Bellow the barbed element 6, there is an elongated shaft 8 of smaller diameter.

The following table shows a process which can be used for manufacturing the micro needle according to the invention.

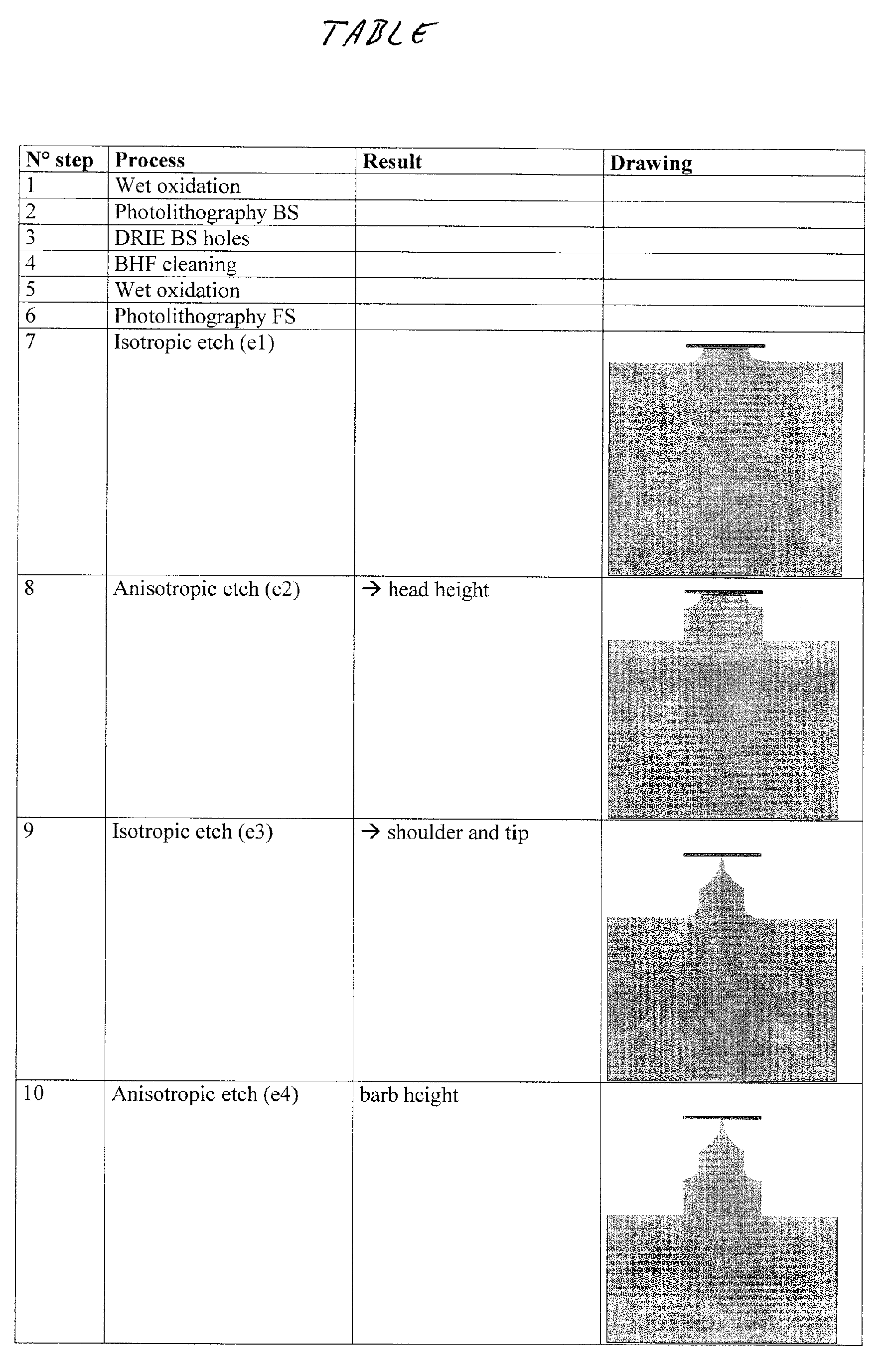

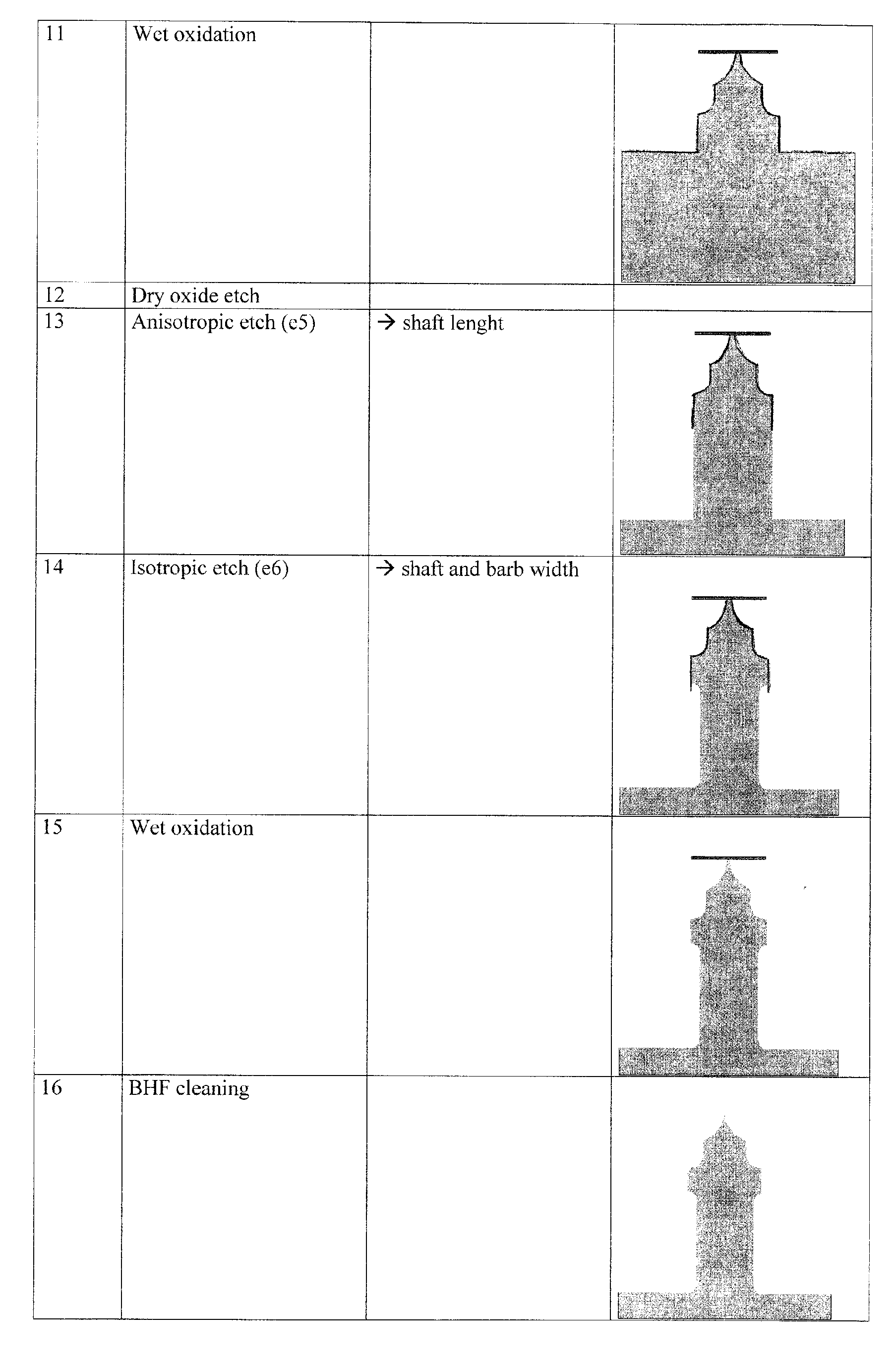

the blind hole and form the needle distal end,
- anistropically etching the protruding structure to form a barbed element having a greater diameter than the diameter of the needle distal end,
- performing a wet oxidation on the needle distal end and on the barbed element followed by a dry oxide etch,
- anistropically etching the protruding structure to form a needle shaft,
- performing a third isotropic etch to form a shaft of smaller diameter than the diameter of the barbed element,
- performing a wet oxidation and a BHF cleaning.

## Claims

1. Hollow out-of-wafer-plane micro needle (1) comprising a support member (2), a closed pointed tip portion (3) and at least one side opening (4) in the needle body communicating with the needle lumen (5), **characterized by** the fact that it comprises at least one barbed element (6) protruding from the needle body.

2. Mirco needle according to claim 1 wherein said barbed element (6) is located between the support member (2) and the side opening (4).

3. Mirco needle according to claim 1 wherein said barbed element (6) is located between the closed pointed tip portion (3) and the side opening (4).

4. Micro needle according to claim 1, 2 or 3 wherein said barbed element (6) is an annular flange surrounding the needle body.

5. Micro needle according to claim 4 wherein the barbed element is tapered towards the needle distal end (7).

6. Micro needle according to claim 4 or 5 wherein the barbed element is not tapered towards the needle proximal end.

7. Micro needle according to anyone of the previous claims wherein the needle has the general shape of a cross exhibiting a plurality of wings, and wherein said side opening is located in the corners of the cross where said wings connect to each other.

8. Micro needle according to anyone of the previous claims wherein the needle has several side openings.

9. Plate for transdermal transfer of liquid comprising an array of micro needles as claimed in anyone of claims 1 to 8 provided on a support member.

10. Hollow out-of-wafer-plane micro needle obtained according to a
process comprising the following steps :
- providing a wafer comprised of an etchable material and having a front side and a back side,
- making a blind hole in said wafer from its back side,
- providing a mask on the front side of the wafer such that the vertical projection of said mask at least partially covers the extension of said hole,
- performing a first isotropic etch under said mask to remove wafer material,
- anisotropically etching the wafer to form a protruding structure,
- performing a second isotropic etch on said protruding structure to expose the blind hole and form the needle distal end,
- anistropically etching the protruding structure to form a barbed element having a greater diameter than the diameter of the needle distal end,
- performing a wet oxidation on the needle distal end and on the barbed element followed by a dry oxide etch,
- anistropically etching the protruding structure to form a needle shaft,
- performing a third isotropic etch to form a shaft of smaller diameter than the diameter of the barbed element,
- performing a wet oxidation and a BHF cleaning.

11. Process for obtaining an hollow out-of-wafer-plane micro needle
**characterized by** the fact that it comprises the following steps :
- providing a wafer comprised of an etchable material and having a front side and a back side,
- making a blind hole in said wafer from its back side,
- providing a mask on the front side of the wafer such that the vertical projection of said mask at least partially covers the extension of said hole,
- performing a first isotropic etch under said mask to remove wafer material,
- anisotropically etching the wafer to form a protruding structure,
- performing a second isotropic etch on said protruding structure to expose
